# EUROPEAN PATENT APPLICATION

(11) **EP 0 990 672 A2**
(43) Date of publication of application: **05.04.2000**
(21) Application number: 99118351.8
(22) Date of filing: 16.09.1999
(51) Int. Cl.: C08G 63/06, C08G 63/08, C08G 63/81, A61L 26/00

(54) **Biodegradable polyester oligomers**

(30) Priority: 01.10.1998 DE 19845152
(71) Applicant: MERCK PATENT GmbH, 64293 Darmstadt (DE)
(72) Inventor: Pfefferle, Hans-Joachim, 64297 Darmstadt (DE); Nies, Berthold, Dr., 64407 Fränkisch-Crumbach (DE); Jürgens, Christian, Dr., 20149 Hamburg (DE)

(57) **Zusammenfassung**

Die Erfindung betrifft bioabbaubare Polyester-Oligomere hergestellt aus Hydroxycarbonsäuren mit 2 bis 10 C-Atomen unter Verwendung von polyfunktionellen Alkoholen (z.B. Glycerin) zur Einstellung des mitteleren Molekülgewichts der Polyester-Oligomere auf Werte von größer gleich 200, die dadurch gekennzeichnet sind, daß diese mit einem definiertem Gehalt an Lösungsmittel versetzt werden. Sie kommen als Adhäsiv zur Fixierung von Wundabdeckungen, Klebe- oder Dichtmittel zum Einsatz.

## Description

Die EP-0290 983 beschreibt körperresorbierbare Wachse aus bei Körpertemperatur zähviskosen bis festen wachsartigen Polyester-Oligomeren der Milchsäure und/oder Glycolsäure, hergestellt unter Mitverwendung von Glycerin als polyfunktionellem Alkohol zur Regelung des mittleren Molekulargewichts der Polyester-Oligomeren auf Werte im Bereich von etwa 200-1500, wobei diese Wachse zusätzlich dadurch gekennzeichnet sind, daß für die Verwendung zur mechanischen Blutstillung an körpereigenem Gewebe, insbesondere Knochen, ihr Gehalt an nicht-reagierten Hydroxycarbonsäuren auf Restgehalte unter 0.5 Gew.-% abgesenkt ist.
Im Rahmen der Optimierung resorbierbarer Wachse an körpereigenem Hartgewebe hat sich jetzt gezeigt, daß durch Versetzen der Polyester-Oligomere mit bestimmten Lösungsmitteln neue vorteilhafte Eigenschaften hervorgerufen werden. Auf diese Weise können hohe Biokompatibilität mit guten Abdichte- und Klebeeigenschaften kombiniert werden.

Gegenstand der vorliegenden Erfindung sind bioabbaubare Polyester-Oligomere hergestellt aus Hydroxycarbonsäuren mit 2 bis 10 C-Atomen unter Verwendung von polyfunktionellem Alkoholen (z.B. Glycerin) zur Einstellung des mitteleren Molekülgewichts auf Werte größer gleich 200, die dadurch gekennzeichnet sind, daß sie mit einem definierten Gehalt an Lösungsmittel versetzt sind. Als Monomere werden Milchsäure, Capronsäure oder Glycolsäure einzeln oder als Mischung verwendet.
Als Lösungsmittel werden Aceton, Ethylacetat, Tetrahydrofuran oder N-Methylpyrrolidon eingesetzt.
In Abhängigkeit vom Verhältnis Glycerin zu Lactid oder Glycolid, vorzugsweise bei einem Verhältnis Glycerin : Lactid bzw. Glycolid von 1:13 bis 1:20, besitzt das Endprodukt nach Verdunsten des Lösungsmittels eine nicht klebende Oberfläche. Es dient somit im medizinisch-pharmazeutischen Bereich zum Abdichten von Wunden.

Variiert man das Verhältnis Glycerin : Lactid bzw. Glycolid durch Verringerung des Lactid-Gehaltes, so bildet das Produkt nach Verdunsten des Lösungsmittels eine dauerhaft klebende Oberfläche aus. Bevorzugte Produkte im Sinne der Erfindung werden erhalten, wenn man auf 1 Mol Glycerin 3 bis 8 mol des Lactid bzw. Glycolid einsetzt. Sie dienen zum Beispiel als Adhäsiv zur Fixierung von Wundabdeckungen bzw. als Klebemittel.

In den folgenden Beispielen sind Ausführungsformen der bioabbaubaren Polyester-Oligomere näher erläutert. Einzelheiten zur Durchführung der Oligomerisierungsreaktionen finden sich in der zitierten Veröffentlichung gemäß EP-0290 983.

### Herstellung von Polyester-Oligomeren als Dichtmittel

### Beispiel 1

Ein Polymer der Zusammensetzung 1 Teil Glycerin zu 18 Teilen DL-Lactid wird unter Erwärmung in Aceton gelöst und auf einen Lösungsmittelgehalt von 25 % eingestellt. Man erhält eine zähviskose, klebrige Masse, die nach Verdunsten des Lösungsmittels eine nicht klebende Oberfläche ausbildet.

### Herstellung von Polyester-Oligomeren als Klebemittel

### Beispiel 2:

Ein Polymer der Zusammensetzung 1 Teil Glycerin zu 6 Teilen L-Lactid wird unter Erwärmung in Aceton gelöst und auf einen Lösungsmittelgehalt von 20 % eingestellt. Man erhält eine zähviskose, klebrige Masse, die nach Verdunsten des Lösungsmittels eine dauerhaft klebende Oberfläche ausbildet.

## Patentansprüche

1. Bioabbaubare Polyester-Oligomere hergestellt aus Hydroxycarbonsäuren mit 2 bis 10 C-Atomen unter Verwendung polyfunktioneller Alkohole zur Einstellung des mittleren Molekülgewichts auf Werte größer gleich 200, gekennzeichnet durch einen definierten Gehalt an Lösungsmitteln.

2. Polyester-Oligomer gemäß Anspruch 1, dadurch gekennzeichnet, daß als Monomere Milchsäure, Capronsäure oder Glycolsäure einzeln oder als Mischung verwendet werden.

3. Polyester-Oligomere gemäß Anspruch 1 und/oder 2, dadurch gekennzeichnet, daß als polyfunktioneller Alkohol Glycerin verwendet wird.

4. Polyester-Oligomere nach einem der Ansprüche 1 bis 3, dadurch gekennzeichnet, daß das Lösungsmittel aus Aceton, Ethylacetat, Tetrahydrofuran oder N-Methylpyrrolidon besteht.

5. Polyester-Oligomere nach einem der Ansprüche 1 bis 4, dadurch gekennzeichnet, daß es nach Verdunsten des Lösungsmittels, in Abhängigkeit vom Verhältnis Glycerin zu Lactid oder Glycolid, eine nicht klebende Oberfläche besitzt.

6. Polyester-Oligomere nach einem der Ansprüche 1 bis 5, dadurch gekennzeichnet, daß es nach Verdunsten des Lösungsmittels, in Abhängigkeit vom Verhältnis Glycerin zu Lactid oder Glycolid, eine dauerhaft klebende Oberfläche besitzt.

7. Verwendung der bioabbaubaren Polyester-Oligomere nach Ansprüchen 1 bis 6 im medizinisch-pharmzeutischen Bereich, insbesondere als Wundbehandlungsmittel.

8. Verwendung der bioabbaubaren Polyester-Oligomere nach Ansprüchen 1 bis 7 als Adhäsiv, Klebe- oder Dichtmittel.

9. Verwendung der bioabbaubaren Polyester-Oligomere nach Ansprüchen 1 bis 6 als Adhäsiv zur Fixierung von Hautersatz oder Wundabdeckungen.
